# EUROPEAN PATENT APPLICATION

(11) **EP 4 497 372 A1**
(43) Date of publication of application: **29.01.2025**
(21) Application number: 23306289.2
(22) Date of filing: 25.07.2023
(51) Int. Cl.: A61B 5/00, A61B 5/024

(54) **SYSTEM AND METHOD FOR OBTAINING A BIOSIGNAL FROM VIDEO SEQUENCE**

(71) Applicant: Quantiq. IO, 92500 Rueil-Malmaison (FR)
(72) Inventor: NIEL, Fabien, 92500 Rueil-Malmaison (FR)
(74) Representative: Icosa

(57) **Abstract**

The invention relates to a system (100) for obtaining a biosignal of a user from a video sequence of at least one portion of a skin surface of the said user, comprising: at least one input (110) configured to receive a video sequence of a skin surface of the user (1; 2) acquired from a camera (111; 211a), at least one processor (120) configured to: apply a mesh on the frames of the video sequence to define regions of interest, compute a raw trace value based on the value of at the least one point in said region of interest; compute a raw trace signal of the video sequence in said region of interest using said raw trace values; raw photoplethysmography (PPG) signals based on the raw trace signals; segment each of the obtained raw PPG signals using a pre-trained machine learning model to obtain a PPG quality signal; compute processed PPG signals based on the respective PPG quality signal and the associated raw PPG signal; compute a global PPG signal of the portion of the skin surface of the user based on the processed PPG signals, and compute said biosignal based on said global PPG signal of the skin surface of the user; and an output configured to provide the biosignal. A corresponding computer-implemented method is also an object of the present invention.

## Description

### FIELD OF INVENTION

The present invention relates to the field of medical informatics, particularly to remote photoplethysmography (rPPG), wherein physiological data is obtained from a video sequence of a user's skin surface. The present invention more particularly relates to a system for obtaining a biosignal of a user from a video sequence of at least one portion of a skin surface of the said user, and a corresponding computer-implemented method.

### BACKGROUND OF INVENTION

rPPG techniques are employed to measure cardiovascular parameters non-invasively by capturing optical variations of the skin due to cardiac activity. Conventional techniques run the entire rPPG pipeline either entirely on the device or on the cloud. Running the pipeline on the device may lead to challenges regarding the protection of the algorithms and limitations on the possible algorithms that can be used depending on the user's device capabilities. Conversely, running the pipeline on the cloud can lead to privacy issues and bandwidth constraints due to the transfer of raw image data to a remote server.

Further, methods transmitting average color values per image over the network for rPPG processing lack the capacity to retain useful information about the distribution of color on the face, which is crucial for isolating the physiological signal of interest from the noise.

Thus, there is a need for a system and computer-implemented method which solve the abovementioned drawbacks.

### SUMMARY

An object of the invention is a system for obtaining a biosignal of a user from a video sequence of at least one portion of a skin surface of the user. This system incorporates machine learning models to handle mesh application and raw PPG signal segmentation. The invention preserves user privacy and reduces computational load and bandwidth requirements for data transmission, ensuring a more reliable rPPG signal and consistent semantics for regions of interest (ROI). The system is designed to compute biosignals like heart rate, oxygen saturation, respiratory rate, heart rate variability, and arterial pressure.

More precisely, the invention relates to a system for obtaining a biosignal of a user from a video sequence of at least one portion of a skin surface of the said user, comprising: at least one input configured to receive a video sequence of a skin surface of the user acquired from a camera, at least one processor configured to: apply a mesh on the frames of the video sequence to define regions of interest on said at least one portion of skin surface, each region of interest comprising at least one point, compute for each frame and for each region of interest, a raw trace value based on the value of at the least one point in said region of interest, for each region of interest, compute, over the video sequence, a raw trace signal of the video sequence in said region of interest using said raw trace values, compute, for each region of interest, raw photoplethysmography (PPG) signals based on the raw trace signals, segment each of the obtained raw PPG signals using a pre-trained machine learning model to obtain a PPG quality signal representative of the quality of the raw PPG signal, compute, for each region of interest, processed PPG signals based on the respective PPG quality signal and the associated raw PPG signal, compute a global PPG signal of the portion of the skin surface of the user based on the processed PPG signals, and compute said biosignal based on said global PPG signal of the skin surface of the user; and an output configured to provide the biosignal.

The claimed system allows for remote, non-contact biosignal monitoring of a user, reducing the need for wearable sensors and enhancing user comfort. The use of video sequences enables dynamic and regular monitoring of a user's health status, providing a real-time health overview. The application of a mesh on video frames for defining regions of interest optimizes the analysis of photoplethysmographic data, improving the accuracy of the captured biosignals. The computation of raw PPG signals and subsequent segmentation using a machine learning model allows for quality assessment of the PPG signal, enhancing reliability of the resulting biosignal. The system's ability to process raw PPG signals into a global PPG signal and subsequently compute a biosignal provides comprehensive health metrics in a single integrated system. The system's output functionality extends its utility by allowing the integration of the processed biosignal into various health monitoring applications, medical devices, or electronic health records.

According to an embodiment, the skin surface of the user may comprise at least part of the face of the user.

According to an embodiment, the mesh may be a dynamic mesh applied on the frames using a pre-trained machine learning model such that the face is tracked and regions of interest are consistent along the video sequence, the machine learning model being trained on a dataset of face images labelled with a specific mesh.

This ensures consistent regions of interest along the video sequence, improving the quality and consistency of the PPG signal. The system ensures tracking consistency across video frames, improving data reliability over time and motion scenarios. It maximizes region of interest relevancy, thereby reducing errors from movement or position change.

According to an embodiment, the pre-trained machine learning model used to segment each of the obtained raw PPG signals is a 1D convolutional neural network, trained on a dataset of labelled PPG signals.

According to an embodiment, the processed PPG signals may be computed by multiplying the PPG quality signals and the corresponding PPG raw signals with each other.

According to an embodiment, the PPG quality signals may be thresholded, and the processed PPG signals are computed by multiplying the obtained thresholded PPG quality signals and the corresponding PPG raw signals with each other.

According to an embodiment, the biosignal may be one of the following: heart rate, oxygen saturation, respiratory rate, heart rate variability, diastolic pressure, systolic pressure, cardiac workload, hemoglobin level, glycemia.

According to an embodiment, the biosignal is heart rate, and the heart rate is computed using frequential analysis of the PPG signal.

The use of frequential analysis of the PPG signal for heart rate computation provides a robust and reliable method for determining the heart rate, contributing to the precision of cardiovascular monitoring.

According to an embodiment, the raw PPG signals may be obtained by filtering the raw trace signals, said filtering being adapted to the biosignal to obtain. The raw PPG signals may also be normalized.

The computation of raw trace signals by filtering, and specifically averaging, the values of the points in the region of interest, provides a robust method to mitigate signal noise and improve signal clarity.

According to an embodiment, the system may comprise a first processor and a second processor distinct and remote from the first processor, wherein the first processor is configured to: apply the mesh on the frames of the video sequence to define regions of interest on said at least one portion of skin surface, compute for each frame and for each region of interest, the raw trace value based on the value of the at least one point in said region of interest, for each region of interest, compute, over the video sequence, the raw trace signal of the video sequence in said region of interest using said raw trace values; wherein the first or the second processor is configured to compute, for each region of interest, the raw PPG signals based on the raw trace signals; and wherein the second processor is configured to: segment each of the received raw PPG signals using a pre-trained machine learning model to obtain the PPG quality signal representative of the quality of the raw PPG signal, compute the processed PPG signals for each region of interest based on the respective PPG quality signals and the associated raw PPG signals, compute the global PPG signal of the skin surface of the user based on the processed PPG signals, and compute said biosignal based on said global PPG signal of the portion of the skin surface of the user.

In an example, the step of computing, for each region of interest, the raw PPG signals based on the raw trace signals, may be performed either by the first or the second processor. The selection of the processor for performing this step may be carried out dynamically, e.g. based on the bandwidth available for data communication between the first and the second processor, and/or based on the computational power available at the first processor. In other words, a computer-implemented method using a system as described above may also comprise a step of selecting the best processor to carry out the step of computing, for each region of interest, the raw PPG signals based on the raw trace signals.

This system allows for better privacy as the image of the skin of the user is never sent to the second remote processor, while reducing computational resources on the first local processor. The division of computational tasks between a first and second processor promotes efficient use of computational resources, improving the speed and reliability of biosignal computation. The remote nature of the second processor allows for scalability and distribution of processing load, enabling the system to handle larger user bases without compromising performance.

According to an embodiment, the system may further comprise a camera to acquire the video sequence. The integration of a camera within the system allows for complete in-situ data acquisition, enhancing the system's convenience and ease of use.

According to an embodiment, the system comprises a first local device including the camera, the input, the first processor and the output, and a second remote device including the second processor. As mentioned, the partition of the system across a local device and a remote device fosters a balance between immediate data acquisition and heavy computational tasks, enhancing overall system efficiency.

According to an embodiment, the first local device may be a smartphone, a tablet or a computer, and the second remote device may be a cloud server. The potential implementation of the system in everyday devices like smartphones, tablets, or computers promotes user accessibility, contributing to better health monitoring and engagement. The utilization of a cloud server as the remote device leverages the advantages of cloud computing, such as scalability and cost-effectiveness, enhancing the system's feasibility for widespread adoption.

According to an embodiment, the video sequence comprises a plurality of channels, and wherein the at least one processor is configured to, for each channel: apply a mesh on the frames of the channel of the video sequence to define regions of interest on said at least one portion of skin surface, compute for each frame of the channel and for each region of interest, a raw trace value based on the value of at least one point in said region of interest, for each region of interest, compute, over the video sequence, a raw trace signal of the channel of the video sequence in said region of interest using said raw trace values, and wherein, the at least one processor is further configured to: compute, for each region of interest, combined raw trace signals by combining the raw trace signals of all channels of the video sequence, and compute, for each region of interest, raw PPG signals based on the combined raw trace signals.

The use of several channels for the video sequence permits to reduce the noise in the acquired and computed signals.

The invention also introduces a computer-implemented method that follows the same steps as the system for obtaining the biosignal.

In particular, the invention relates to a computer-implemented method (300) for obtaining a biosignal of a user from a video sequence of at least one portion of a skin surface of the said user, comprising the following steps: receive a video sequence of a skin surface of the user, apply a mesh on the frames of the video sequence to define regions of interest on said at least one portion of skin surface, each region of interest comprising at least one point, compute for each frame and for each region of interest, a raw trace value based on the value of at least one point in said region of interest, for each region of interest, compute, over the video sequence, a raw trace signal of the video sequence in said region of interest using said raw trace values, compute, for each region of interest, raw PPG signals based on the raw trace signals, segment each of the obtained raw PPG signals using a pre-trained machine learning model to obtain a PPG quality signal representative of the quality of the raw PPG signal, compute, for each region of interest, processed PPG signal based on the respective PPG quality signal and the associated raw PPG signal, compute a global PPG signal of the portion of the skin surface of the user based on the processed PPG signals, and compute said biosignal based on said global PPG signal of the skin surface of the user

The method implements non-invasive, real-time, and remote biosignal monitoring, which can be integrated with various devices and applications, promoting user engagement and continuity of health monitoring. The application of machine learning techniques in the method allows for the automation and optimization of signal quality assessment and biosignal computation, enhancing the accuracy and reliability of the obtained biosignals.

Further embodiments of the invention include a computer program and a computer-readable medium containing instructions for carrying out the steps of the method.

### DEFINITIONS

As used in the context of the present invention, the term **"machine learning (ML) model"** refers to a computational algorithm or a set of algorithms that are capable of learning patterns or relationships in input data through a training process. The learned patterns or relationships can then be used to make predictions, classifications, or recommendations for new, previously unseen data. Machine learning models can be based on various techniques, including but not limited to supervised learning, unsupervised learning, semi-supervised learning, reinforcement learning, and deep learning. Examples of machine learning models include, but are not limited to, decision trees, support vector machines, artificial neural networks, k-means clustering, principal component analysis, and reinforcement learning agents. In the context of this invention, machine learning models are used to segment raw PPG signals and, advantageously, to apply a dynamic mesh to define regions of interest.

A **"hyper-parameter"** presently means a parameter used to carry out an upstream control of a model construction, such as a remembering-forgetting balance in sample selection or a width of a time window, by contrast with a parameter of a model itself, which depends on specific situations. In ML applications, hyper-parameters are used to control the learning process.

**"Datasets"** are collections of data used to build an ML mathematical model, so as to make data-driven predictions or decisions. In **"supervised learning"** (i.e. inferring functions from known input-output examples in the form of labelled training data), three types of **ML** datasets (also designated as **ML** sets) are typically dedicated to three respective kinds of tasks: **"training",** i.e. fitting the parameters, **"validation",** i.e. tuning ML hyperparameters (which are parameters used to control the learning process), and **"testing",** i.e. checking independently of a training dataset exploited for building a mathematical model that the latter model provides satisfying results.

A **"neural network (NN)"** designates a category of **ML** comprising nodes (called **"neurons"),** and connections between neurons modeled by **"weights".** For each neuron, an output is given in function of an input or a set of inputs by an **"activation function".** Neurons are generally organized into multiple **"layers",** so that neurons of one layer connect only to neurons of the immediately preceding and immediately following layers.

The above ML definitions are compliant with their usual meaning, and can be completed with numerous associated features and properties, and definitions of related numerical objects, well known to a person skilled in the ML field. Additional terms will be defined, specified or commented wherever useful throughout the following description.

The terms **"adapted", "designed"** and **"configured"** are used in the present disclosure as broadly encompassing initial configuration, later adaptation or complementation of the present device, or any combination thereof alike, whether effected through material or software means (including firmware).

The term **"processor"** should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

### BRIEF DESCRIPTION OF THE DRAWINGS

The present disclosure will be better understood, and other specific features and advantages will emerge upon reading the following description of particular and non-restrictive illustrative embodiments, the description making reference to the annexed drawings wherein:
**Figure 1** illustrates a system according to an embodiment of the invention.
**Figure 2** illustrates a system according to another embodiment of the invention.
**Figure 3** details a flowchart representing the steps of a computer-implemented method according to an embodiment of the invention.
**Figure 4** shows an example of mesh applied to a frame containing the face of a user.
**Figure 5** exemplifies the segmentation of a raw PPG signal by a pre-trained machine learning model.
**Figure 6** shows a comparative graph of the heart rate obtained by a method or system according to the invention and a heart rate obtained by a reference oximeter.

### DETAILED DESCRIPTION

The present description illustrates the principles of the present disclosure. It will thus be appreciated that those skilled in the art will be able to devise various arrangements that, although not explicitly described or shown herein, embody the principles of the disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

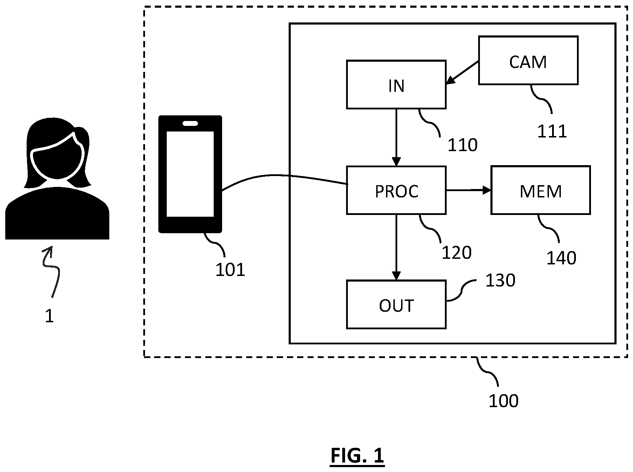

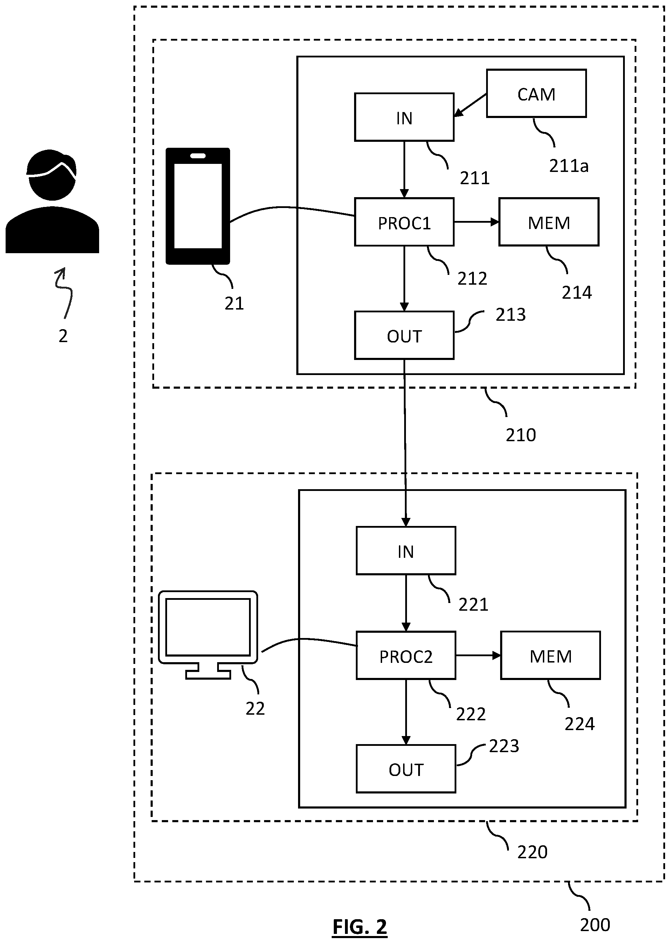

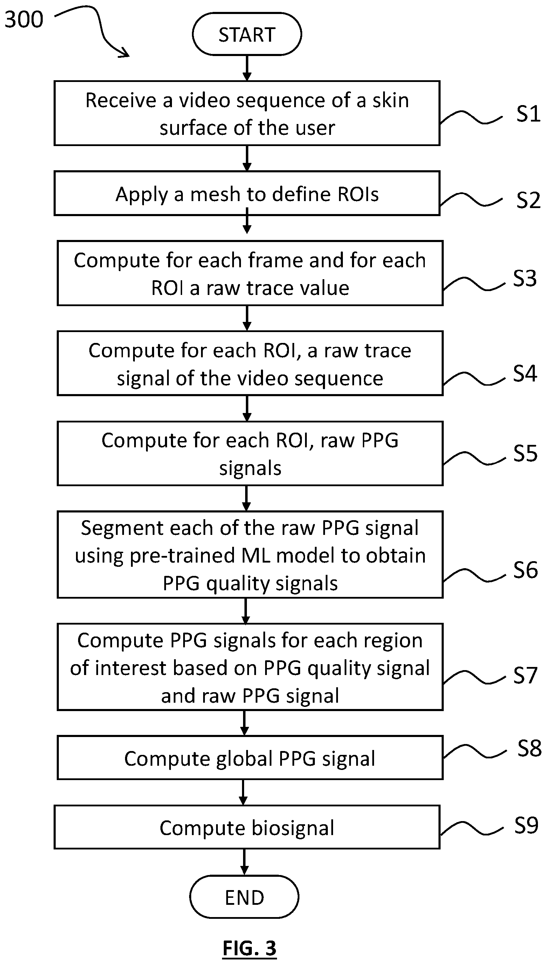

The invention thus relates to a system for obtaining a biosignal of a user from a video sequence of at least one portion of a skin surface of the said user, as shown in Figures 1 and 2, and a corresponding computer-implemented method as shown in Figure 3. The system may be a remote photoplethysmography system.

### SYSTEM

In the first example shown in **figure 1****,** the system 100 generally comprises an input 110 configured to receive a video sequence or stream of at least a portion of a skin surface of the user 1, a processor 120 configured to process the video sequence and obtain the biosignal, and an output 130 configured to output the biosignal. It may also comprise a camera 111 to provide the video sequence to the input 110, and a memory 140.

The biosignal may be one of the following: heart rate, oxygen saturation, respiratory rate, heart rate variability, diastolic pressure, systolic pressure, cardiac workload, hemoglobin level, glycemia.

In this example, the system 100 may be integrated in a single computer (e.g. in a telemedicine cabin booth), smartphone 101 or tablet. Other devices may also be contemplated.

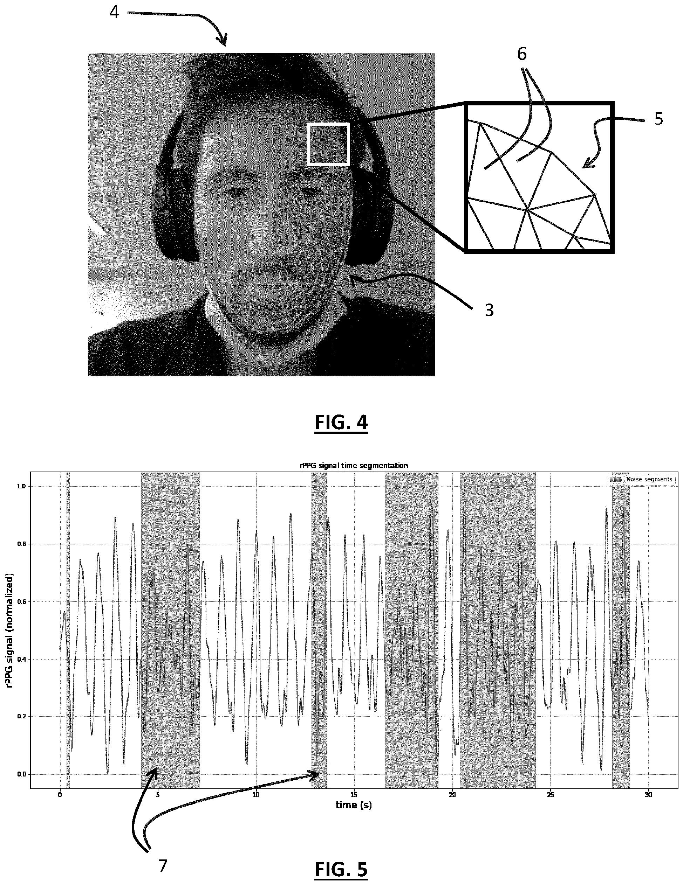

The input 110 is configured to receive the video sequence of at least a portion of a skin surface of the user 2 acquired from the camera. The video sequence comprises a plurality of images or frames. An example of image or frame 3 from a video sequence captured from a camera is shown at figure 4. Preferably, the portion of the skin surface comprises at least part of the face 3 of the user.

The camera 111 may be a single-color camera, or multi-channel camera, like a RGB camera. If the camera is a multi-channel camera, the video sequence then comprises as many channels.

The processor is configured to perform the following steps, which will be explained in greater details thereafter with respect to figure 3 and the computer-implemented method:
a. Step S2: apply a mesh 5 (figure 4) on the frames 4 of the video sequence to define regions of interest 6 on said at least one portion of skin surface,
b. Step S3: compute for each frame and for each region of interest, a raw trace value based on the value of at least one point in said region of interest,
c. Step S4: for each region of interest, compute, over the video sequence, a raw trace signal of the video sequence in said region of interest using said raw trace values,
d. Step S5: for each region of interest, compute raw photoplethysmography (PPG) signals based on the raw trace signals,
e. Step S6: segment each of the obtained raw PPG signals using a pre-trained machine learning model to obtain a PPG quality signal representative of the quality of the raw PPG signal,
f. Step S7: compute processed PPG signals for each region of interest based on the respective PPG quality signal and the associated raw PPG signal,
g. Step S8: compute a global PPG signal of the skin surface of the user based on the processed PPG signals, and
h. Step S9: compute said biosignal based on said global PPG signal of the skin surface of the user.

The output 130 is configured to provide the biosignal thus computed.

The memory 140 may be used to store the video sequence, signals and data necessary to compute the biosignal (e.g. machine learning models parameters, etc.). The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk).

The system 100 may further comprise a display to display the biosignal provided by the output 130.

The system 100 may further comprise communication means to wirelessly communicate with remote devices, for example to transmit the biosignal to another device for remote health monitoring.

In the advantageous embodiment shown in **figure 2****,** the system 200 comprises two subsystems (or devices) 210 and 220. In this example, the first subsystem 210 comprises a first input 211, a processor 212, an output 213, a camera 211a and a memory 214. In this example, the second subsystem 200 comprises a second input 221, a second processor 222, a second output 223 and a second memory 224.

In this example, the first processor 212 is configured to:
a. apply the mesh on the frames of the video sequence received at the first input 211 (e.g. from the camera 211a) to define regions of interest on said at least one portion of skin surface,
b. compute for each frame and for each region of interest, the raw trace value based on the value of at least one point in said region of interest,
c. for each region of interest, compute, over the video sequence, the raw trace signal of the video sequence in said region of interest using said raw trace values,
d. compute, for each region of interest, the raw PPG signals based on the raw trace signals, and
   send the raw PPG signals thus obtained to the second processor 222 (via the second input 221); and
wherein the second processor 222 is configured to:
e. segment each of the received raw PPG signals using a pre-trained machine learning model to obtain a PPG quality signal representative of the quality of the raw PPG signal,
f. compute processed PPG signals for each region of interest based on the respective PPG quality signal and the associated raw PPG signal,
g. compute the global PPG signal of the skin surface of the user based on the processed PPG signals, and
h. compute said biosignal based on said global PPG signal of the skin surface of the user.

It should be noted that the step of computing, for each region of interest, the raw PPG signals based on the raw trace signals, may be performed either by the first 212 or the second 222 processor. The selection of the processor for performing this step may be carried out dynamically, e.g. based on the bandwidth available to communicate data between the first and the second processor, and/or based on the computational power available at the first processor.

The functions of memories 214 and 224 are similar as in system 100.

The subsystem 210 may comprise a first local device 21 (e.g. a smartphone, a tablet or a computer), including the first input 211, the camera 211a, the first processor 212, the first output 213 and the memory 214. The subsystem 220 may comprise a second remote device 22 (e.g. a cloud server), including the second input 221, the second processor 222, the second output 223 and the second memory 224.

In this example, the second output 223 may not be used, and the second processor 222 may send back the computed biosignal to the first processor 212 such that the first output 213 provides the biosignal at the first subsystem 210.

This example is advantageous as it increases privacy (no image of the subject is directly sent to a remote device), while distributing computation load between the first device 21 (which may have low computation capabilities, like a smartphone or tablet), and the second device 22 (which may have greater computation capabilities, like a cloud server).

### COMPUTER-IMPLEMENTED METHOD

A computer-implemented method for obtaining a biosignal of a user from a video sequence of at least one portion of a skin surface of the said user according to an embodiment of the invention will be described in greater details with respect to figures 3, 4 and 5. The biosignal may be one of the following: heart rate, oxygen saturation, respiratory rate, heart rate variability, diastolic pressure, systolic pressure, cardiac workload, hemoglobin level, glycemia.

As shown in **figure 3****,** the method comprises at least the following steps:

At step S 1, a video sequence of a skin surface of the user acquired from a camera is received.

Then, at step S2 a mesh 5 is applied on the frames 4 of the video sequence to define regions of interest 6 on said at least one portion of skin surface. The skin surface of the user advantageously comprises at least part of the face 3 of the user.

In an embodiment, the mesh is a dynamic mesh applied on the frames using a pre-trained machine learning model such that the face is tracked and regions of interest are consistent along the video sequence, the machine learning model being trained on a dataset of face images labelled with a specific mesh. Said machine learning model may be for example a 2D convolutional neural network, e.g. ResNet.

Then, at step S3, for each frame and for each region of interest, a raw trace value is computed based on the value of at least one point in said region of interest. The raw trace signals may be computed by filtering the values of the points of the region of interest, preferably by averaging the values of the points in the region of interest. Other methods of filtering may be used, e.g. using colour filtering, a machine learning model for segmentation or classification, thresholding, etc.

Then, at step S4, for each region of interest 6, and over the video sequence, a raw trace signal of the video sequence in said region of interest is computed using said raw trace values. This raw trace signal may be the concatenation of the raw trace values.

Then, at step S5, for each region of interest, raw photoplethysmography (PPG) signals based on the raw trace signals are computed.

If only one channel is used for the video sequence, the raw PPG signals may be obtained by normalizing and/or further filtering the raw trace signals depending on the biosignal to obtain. For example, for heart rate, the signal may be bandpass filtered between 0,6 Hz and 4 Hz.

If several channels are used for the video sequence (e.g. when a RGB camera is used, 3 channels are available), the method may further comprise a step of computing, for each region of interest, combined raw trace signals by combining the raw trace signals of all channels of the video sequence, and a step of computing for each region of interest, raw PPG signals based on the combined raw trace signals. Raw trace signals of all channels may be combined by using appropriate models to better extract relevant information for the considered biosignal. Again, raw PPG signals may be obtained by normalizing and/or further filtering the raw trace signals based on the biosignal to obtain.

The raw PPG signals may further be normalized and/or filtered before step S6.

Then, at step S6, each of the obtained raw PPG signals are segmented using a pre-trained machine learning model to obtain a PPG quality signal representative of the quality of the raw PPG signal. For example, the pre-trained machine learning model used to segment each of the obtained raw PPG signals is a 1D convolutional neural network (e.g. Unet), trained on a dataset of labelled PPG signals. The method may comprise a step of training the said machine learning model.

**Figure 5** shows an example of raw PPG signal segmentation by a pre-trained machine learning model. In this example, the model outputs a binary value (0 or 1). Bad quality zones 7 (output value = 0) are identified in grey along the 30 seconds sequence shown in the figure. In other examples, the model may output continuous values between 0 and 1.

Then, at step S7, processed PPG signals are computed for each region of interest based on the respective PPG quality signal and the associated raw PPG signal.

In an example, the PPG quality signals may thresholded (e.g. the PPG quality signal can only take values 0 or 1), and the processed PPG signals may be computed by multiplying the obtained thresholded PPG quality signals and the corresponding PPG raw signals with each other. Other types of computations may be used to obtain the processed PPG signals, for example using a machine learning model trained to reconstruct a signal based on the PPG quality signal and the raw PPG signal.

Then, at step S8, a global PPG signal of the skin surface of the user is computed based on the processed PPG signals. The global PPG signal may be computed by selecting the best quality processed PPG signal, or by combining best quality parts of the processed PPG signals, or using a machine learning model to predict the global PPG signal based on the processed PPG signals.

Finally, at step S9, said biosignal is computed based on said global PPG signal of the skin surface of the user.

In an example, the biosignal is heart rate, and the heart rate is obtained by frequential analysis of the global PPG signal.

It should be noted, that other metrics may be obtained using the system 100 or 200 or using a computer-implemented method 300 as described above, based on obtained biosignals, for example: irregular heartbeats events, atrial fibrillation events, cardiac workload, mental stress index, anxiety index, depression index, cardiovascular disease risk, heart attack risk, stroke risk, hypertension risk, type 2 diabetes risk, hypercholesterolemia risk, hyperglyceridemia risk, etc.

The above computer-implemented method may be implemented in systems 100 or 200.

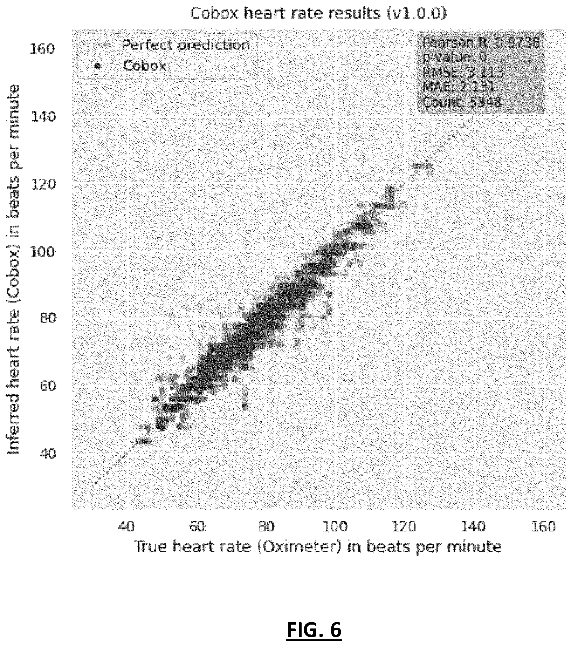

**Figure 6** shows a comparison plot of heart rate obtained by a system according to the invention implementing a method according to the invention for obtaining heart rate using a RGB camera, and a reference certified medical device. These measurements were obtained during a clinical study at hospital emergencies on various different patients. Moreover, the measurements were carried out by physicians and nurses without specific formation. One can see that the invention provides very good match for heart rate measurement in such conditions.

The present disclosure also relates to a computer program product. The computer program product includes instructions which, when the program is run by a computer, cause the computer to automatically perform the steps of the method 300 according to one of the embodiments described above.

The computer program product enabling the execution of the method 300 described above can be written as computer programs, code segments, instructions, or any combination thereof, to individually or collectively instruct or configure the processor or the computer to function as a specific use machine or computer to perform the operations carried out by hardware components. In one example, the computer program comprises machine code directly executed by a processor or computer, such as machine code produced by a compiler. In another example, the computer program product comprises high-level code which is executed by a processor or computer using an interpreter. Programmers with ordinary skills in the art can easily write the instructions or software based on the functional schemes and flow charts illustrated in the drawings and corresponding descriptions in the specification, which disclose algorithms to perform the operations of the method as described above.

## Claims

1. A system (100; 200) for obtaining a biosignal of a user from a video sequence of at least one portion of a skin surface of the said user, comprising:
- at least one input (110; 211) configured to receive a video sequence of a skin surface of the user (1; 2) acquired from a camera (111; 211a),
- at least one processor (120; 212, 222) configured to:
a) apply a mesh (5) on the frames (4) of the video sequence to define regions of interest (6) on said at least one portion of skin surface, each region of interest comprising at least one point,
b) compute for each frame and for each region of interest, a raw trace value based on the value of at the least one point in said region of interest,
c) for each region of interest, compute, over the video sequence, a raw trace signal of the video sequence in said region of interest using said raw trace values,
d) compute, for each region of interest, raw photoplethysmography (PPG) signals based on the raw trace signals,
e) segment each of the obtained raw PPG signals using a pre-trained machine learning model to obtain a PPG quality signal representative of the quality of the raw PPG signal,
f) compute, for each region of interest, processed PPG signals based on the respective PPG quality signal and the associated raw PPG signal,
g) compute a global PPG signal of the portion of the skin surface of the user based on the processed PPG signals, and
h) compute said biosignal based on said global PPG signal of the skin surface of the user; and
- an output configured to provide the biosignal.

2. The system according to claim 1, wherein the skin surface of the user comprises at least part of the face (3) of the user.

3. The system according to claim 2, wherein the mesh (5) is a dynamic mesh applied on the frames using a pre-trained machine learning model such that the face is tracked and regions of interest are consistent along the video sequence, the machine learning model being trained on a dataset of face images labelled with a specific mesh.

4. The system according to any one of claims 1 to 3, wherein the pre-trained machine learning model used to segment each of the obtained raw PPG signals is a 1D convolutional neural network, trained on a dataset of labelled PPG signals.

5. The system according to any one of claims 1 to 4, wherein the raw trace signals are computed by filtering the values of the points of the region of interest, preferably by averaging the values of the points in the region of interest (6).

6. The system according to any one of claims 1 to 5, wherein the PPG quality signals are thresholded, and the processed PPG signals are computed by multiplying the obtained thresholded PPG quality signals and the corresponding PPG raw signals with each other.

7. The system according to any one of claims 1 to 6, wherein the biosignal is one of the following: heart rate, oxygen saturation, respiratory rate, heart rate variability, diastolic pressure, systolic pressure, cardiac workload, hemoglobin level, glycemia.

8. The system according to claim 7, wherein the biosignal is heart rate, and the heart rate is computed using frequential analysis of the global PPG signal.

9. The system according to any one of claims 1 to 8, wherein the raw PPG signals are obtained by filtering the raw trace signals, said filtering being adapted to the biosignal to obtain.

10. The system (200) according to any one of claims 1 to 9, comprising a first processor (212) and a second processor (222) distinct and remote from the first processor, wherein the first processor (212) is configured to:
a) apply the mesh (6) on the frames (4) of the video sequence to define regions of interest (6) on said at least one portion of skin surface,
b) compute for each frame and for each region of interest, the raw trace value based on the value of the at least one point in said region of interest,
c) for each region of interest, compute, over the video sequence, the raw trace signal of the video sequence in said region of interest using said raw trace values,
wherein the first (212) or the second (222) processor is configured to:
d) compute, for each region of interest, the raw PPG signals based on the raw trace signals; and
wherein the second processor (222) is configured to:
e) segment each of the raw PPG signals using a pre-trained machine learning model to obtain the PPG quality signal representative of the quality of the raw PPG signal,
f) compute the processed PPG signals for each region of interest based on the respective PPG quality signals and the associated raw PPG signals,
g) compute the global PPG signal of the skin surface of the user based on the processed PPG signals, and
h) compute said biosignal based on said global PPG signal of the portion of the skin surface of the user.

11. The system according to any one of claims 1 to 10, further comprising a camera (111; 211a) to acquire the video sequence.

12. The system according to claim 11, comprising a first local device (210) including the camera, the input, the first processor and the output, and a second remote device (220) including the second processor.

13. The system according to any one of claims 10 to 12, wherein the first local device is a smartphone (21), a tablet or a computer, and the second remote device is a cloud server (22).

14. The system according to any one of claims 1 to 13, wherein the video sequence comprises a plurality of channels, and wherein the at least one processor (120; 212, 222) is configured to, for each channel:
a) apply a mesh on the frames of the channel of the video sequence to define regions of interest on said at least one portion of skin surface,
b) compute for each frame of the channel and for each region of interest, a raw trace value based on the value of at least one point in said region of interest,
c) for each region of interest, compute, over the video sequence, a raw trace signal of the channel of the video sequence in said region of interest using said raw trace values,
and wherein, the at least one processor is further configured to:
c2) compute, for each region of interest, combined raw trace signals by combining the raw trace signals of all channels of the video sequence, and
d) compute, for each region of interest, raw PPG signals based on the combined raw trace signals.

15. A computer-implemented method (300) for obtaining a biosignal of a user from a video sequence of at least one portion of a skin surface of the said user, comprising the following steps:
- receive a video sequence of a skin surface of the user (S1),
- apply a mesh on the frames of the video sequence to define regions of interest (S2) on said at least one portion of skin surface, each region of interest comprising at least one point,
- compute for each frame and for each region of interest, a raw trace value (S3) based on the value of at least one point in said region of interest,
- for each region of interest, compute, over the video sequence, a raw trace signal of the video sequence (S4) in said region of interest using said raw trace values,
- compute, for each region of interest, raw PPG signals (S5) based on the raw trace signals,
- segment each of the obtained raw PPG signals (S6) using a pre-trained machine learning model to obtain a PPG quality signal representative of the quality of the raw PPG signal,
- compute, for each region of interest, processed PPG signal (S7) based on the respective PPG quality signal and the associated raw PPG signal,
- compute a global PPG signal (S8) of the portion of the skin surface of the user based on the processed PPG signals, and
- compute said biosignal (S9) based on said global PPG signal of the skin surface of the user.
